(19) Europäisches Patentamt
European Patent Office
Office européen des brevets

(11) **EP 4 338 776 A1**

(12) **EUROPEAN PATENT APPLICATION**

(43) Date of publication:
**20.03.2024 Bulletin 2024/12**

(21) Application number: **22206553.4**

(22) Date of filing: **10.11.2022**

(51) International Patent Classification (IPC):
***A61M 16/00*** *(2006.01)*

(52) Cooperative Patent Classification (CPC):
**A61M 16/024;** A61M 2205/13; A61M 2205/3344;
A61M 2230/005; A61M 2230/42

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **19.09.2022 PCT/CN2022/119713**

(71) Applicant: **Koninklijke Philips N.V.
5656 AG Eindhoven (NL)**

(72) Inventors:
• **FU, Junzeng
Eindhoven (NL)**
• **SHI, Jun
Eindhoven (NL)**
• **YIN, Bin
5656AG Eindhoven (NL)**

(74) Representative: **Philips Intellectual Property &
Standards
High Tech Campus 52
5656 AG Eindhoven (NL)**

(54) **POSITIVE AIRWAY PRESSURE SUPPORT SYSTEMS**

(57) A control system is provided for controlling a pressure applied by a pressure source of a positive airway pressure support system. A progressive increase is provided in the pressure applied during a fall asleep time period of a user of the system. A pressure modulation is applied during the progressive increase. The modulation is for promoting a target breathing rate of the user,

FIG. 1

EP 4 338 776 A1

## Description

FIELD OF THE INVENTION

This invention relates to positive airway pressure support systems, and in particular it relates to systems which provide a progressive increase in pressure while a user is going to sleep.

BACKGROUND OF THE INVENTION

Positive airway pressure (PAP) is first-line therapy for patients with moderate to severe obstructive sleep apnea (OSA), and also a recommended intervention for chronic hypercapnic chronic obstructive pulmonary disease (COPD). Obstructive sleep apnea and insomnia are the two most common clinical conditions seen in an adult sleep clinic. Of the subtypes of insomnia, sleep maintenance insomnia (waking up often during the night) is found to be the most common type among patients with OSA, and the prevalence of onset insomnia is similar to the general population. The prevalence of insomnia is increased in patients with COPD.

A study has found that PAP treatment can significantly reduce symptoms of sleep-maintenance insomnia, but not for symptoms of sleep-onset insomnia. Sleep-onset insomnia may negatively affect PAP adherence because patients are awake longer and thus more likely to experience the adverse aspects of this treatment (e.g., mask or airflow discomfort) for longer periods of time.

Various features of PAP treatment systems are known, for improving the comfort during the PAP therapy, and ultimately improve the total user experience. For example, the use of a ramp pressure profile is commonly used to increase the air pressure incrementally over a fixed period of time to allow the patient to fall asleep before the higher pressure settings (e.g. therapeutic pressures) are engaged.

The ramp delivers pressurized air from a low setting, gradually "ramping" up to the set pressure. The ramp function allows patients to feel comfortable breathing against pressurized air while falling asleep. With a ramp function, the PAP therapy starts from low pressure (e.g., 4 cmH2O), then slowly increases to the pre-set pressure (i.e., treatment pressure) during a pre-defined time (e.g., <45 min). However, for some PAP users with on-set insomnia, it is still challenging to fall asleep.

There remains a need for a PAP system to enable users to fall asleep more comfortably.

SUMMARY OF THE INVENTION

The invention is defined by the claims.

According to examples in accordance with an aspect of the invention, there is provided a control system for controlling a pressure applied by a pressure source of a positive airway pressure support system, wherein the control system comprises a controller which is configured:

to apply a progressive increase in the pressure applied during a fall asleep time period of a user of the system; and
to apply a pressure modulation during the progressive increase, wherein the modulation is for promoting a target breathing rate of the user.

This control system uses the pressure source of a PAP support system to implement a slow-paced breathing (SPB) guidance function (i.e., setting a target breathing rate which the user tries to follow), during the time when a user is trying to fall asleep. The pressure modulation means the pressure toggles between higher and lower values to correspond to target inhalation and exhalation timings, superposed over the progressive increase (e.g., ramp) in the pressure applied.

Slow-paced breathing is a breathing technique that aims to slow down the inhalation and exhalation phases to a controlled pace that is determined by a visual, auditory, or kinesthetic pacer. Spontaneous breathing usually comprises between 12 and 20 breaths per minute (bpm) in adults. It is reported that SPB, for example at 6 bpm, has positive effects on sleep quality and emotional well-being. The underlying mechanism can be explained as SPB enhancing respiratory sinus arrhythmia, vagal afferents within the inhalation phase via stretching of the pulmonary afferents and the vagal afferents in both the short and long term.

Thus, by promoting a target (low) breathing rate, sleep quality is improved, and users are assisted in falling asleep. Thus, it is of particular interest for patients having PAP support who suffer from sleep-onset insomnia. The slow-paced breathing function takes place during a ramp period of the pressure support, which is also used to assist users in falling asleep, by reducing the discomfort of the PAP support when they are initially going to sleep. It also makes use of the pressure source of the PAP system to provide the pacing mechanism for the slow-paced breathing. Additional pacing mechanisms may however additionally be provided, such as audio or tactile instructions for slow paced breathing.

The progressive increase in pressure (e.g., ramp) for example increases from a first pressure (e.g., 4cmH2O = 400Pa) to a treatment pressure (e.g., 10cmH20 = 1kPa). Different treatment pressures may be used and the treatment pressure is for example settable at different levels for different users. The target breathing rate is for example a low breathing rate such as around 6 bpm.

The controller is for example further configured to receive a current breathing rate of the user from a breathing rate sensor.

This is used as a feedback parameter, for example to control the level of the target breathing rate to make it appropriate to the user's initial breathing rate and/or their current breathing rate, and to monitor if the user is able to attain their currently set target breathing rate. The slow-paced breathing function is for example not needed if the current breathing rate is already below the desired breathing rate. Thus, the system may have an operating mode whereby the slow-paced breathing function (the pressure modulation) is not used.

The controller is for example configured to apply a pressure modulation for promoting a target breathing rate which depends on the current breathing rate of the user. Thus, the target breathing rate can be made achievable for the user, because it takes into account their current breathing rate.

There is for example a desired breathing rate to be reached by the end of the progressive increase in the pressure applied, and the controller is configured:

in a first mode, if the current breathing rate is below a first threshold (A) and above the desired breathing rate, to set the desired breathing rate as the target breathing rate; and
in a second mode if the current breathing rate is above a threshold, to set the target breathing rate to a target above the desired breathing rate and to change to the first mode when the current breathing rate reaches the target.

If the user has a breathing rate sufficiently close to the desired breathing rate, the desired slow-paced breathing rate can be the target straight away. If the user has a higher breathing rate, an additional higher breathing rate target is set and a subsequent reduction from the additional higher breathing rate target to the desired breathing rate would be necessary.

The second mode may comprise a set of thresholds, wherein if the current breathing rate is above a highest threshold, the controller is configured to set the target breathing rate to a highest target, to change to a lower target when the current breathing rate reaches the current target, and to change to the first mode when the current breathing rate reaches a lowest target of the second mode.

There may be one or more steps from a highest target, to one or more intermediate targets, before the desired end target (i.e. the desired breathing rate) is reached. In one example there are two targets before the desired breathing rate.

The one or more target breathing rates are for example set in dependence on an initial or default breathing rate of the user. The target breathing rate or rates may evolve over time to make a progression to a desired slow-paced breathing rate more easily achievable for a particular user.

The controller is for example configured in a third mode, if the user cannot follow the one or more targets of the second mode and is still awake, to reduce the target breathing rate progressively and/or gradually from a starting value towards to the desired breathing rate. This will take place if the second mode has been applied for a predetermined length of time.

The starting value in the third mode for example comprises:

if the current breathing rate is below the first threshold and above the desired breathing rate, a target ($\beta 3$) which is a function of the desired breathing rate ($\alpha$) and the breathing rate of the user and is greater than the desired breathing rate; and
if the current breathing rate is above the first threshold, the target above the desired breathing rate of the second mode.

The target $\beta 3$ is for example:

$$\beta 3 = \alpha + a \times (BR\_user - \alpha)$$

Thus, the same target may be used as in the second mode if the breathing rate is above the first threshold. However, if the breathing rate is already below the first threshold, a new starting target is set (not immediately equal to the desired breathing rate as is the case for the first mode).

The gradual reduction may for example be a reduction in the target breathing rate at a fixed rate of change (e.g., a change of 0.5 bpm/minute). In one embodiment, the target breathing rate can be reduced gradually to the desired breathing rate. This will make the user more comfortable in terms of changes in breathing rate.

The controller is for example configured to determine a difference between the current breathing rate of the user and the target breathing rate thereby to determine a measure of how well the user can follow the target breathing rate, and:

before a time period threshold has been reached, if the user cannot follow the target breathing rate sufficiently well and has not fallen asleep, to end the pressure modulation; and
before a time period threshold has been reached, if the user can follow the target breathing rate sufficiently well but has not fallen asleep, to continue the pressure modulation; and
to end the pressure modulation when the time period threshold has been reached or after the user has fallen asleep.

Once the user has followed the target breathing rate with the assistance of the slow-paced breathing function, the slow-paced breathing function may be continued until the user falls sleep (and hence can no longer follow the guidance), or the slow-paced breathing function may cease after a pre-set time. If the user cannot follow the target breathing rate, the pressure modulation may also be ended (because it is not working for that user), and a different mode may be used. The pressure modulation may be ended as soon as the user is detected as having fallen asleep or a fixed time afterwards.

The controller is for example configured to implement:

a first overall system mode without the progressive increase;
a second overall system mode with the progressive increase but without the pressure modulation; and
a third overall system mode with the progressive increase and with the pressure modulation.

The control system can thus be used for different users in different ways, depending on their need or desire for a pressure ramp, and their need for slow-paced breathing assistance. Thus, the controller can perform conventional control approaches as well as the new approach of the invention.

The controller is for example configured to select one or more target breathing rates for the pressure modulation (for example an initial target) in dependence on a category of user, wherein the category relates to a default breathing rate of the user. Thus, the target breathing rate may start at a level suitable for the particular user, and the user is defined by a category (e.g., fast breather, medium breather, slow breather).

The default breathing rate is for example sensed at the start of use of the system and the category of user is derived from the sensed breathing rate.

The pressure modulation may comprise a first pressure for an inhalation period of a breathing cycle and a drop from the first pressure to a second pressure for an exhalation period of the breathing cycle.

The modulation for example generates an inhalation pressure which follows the progressive pressure increase (i.e. ramp), and a lower exhalation pressure.

The modulation for example provides a drop in pressure, e.g., 1 to 2 cmH2O for exhalation. The amount of pressure drop may be fixed or it may depend on the underlying ramp pressure, so it may for example be greater when the ramp pressure is greater.

The ramp pressure may be a smooth monotonous increase in pressure, or it may take place in steps.

The invention also provides a positive airway pressure support system, comprising:

a pressure source; and
the control system as defined above for controlling a pressure applied by the pressure source.

The invention also provides a method of controlling a pressure applied by a pressure source of a positive airway pressure support system, comprising:

applying a progressive increase in the pressure applied during a fall asleep time period of a user of the system; and

applying a pressure modulation during the progressive increase, wherein the modulation is for promoting a target breathing rate of the user.

The invention also provides a computer program comprising computer program code means which is adapted, when said program is run on the controller of the positive airway pressure support system defined above, to implement the method defined above.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a graph of pressure applied by a positive airway pressure (PAP) system over time, using a most basic implementation of the invention;
Fig. 2 shows a graph of pressure applied by a positive airway pressure (PAP) system over time, using a more sophisticated implementation of the invention;
Fig. 3 shows a first part of a flow chart to explain the operation of the system pressure control system;
Fig. 4 shows different overall system modes that may be enabled, as a flow chart which follows from Fig. 3;
Fig. 5 shows a first part of a flow chart to explain the different steps of the slow-paced breathing function;
Fig. 6 shows how it is determined when to stop the slow-paced breathing guidance for situations where the user has or has not fallen asleep;
Fig. 7 shows breathing rate categories;
Fig. 8 shows an example of the user breathing rate and the target breathing rate for a first scenario;
Fig. 9 shows an example of the user breathing rate and the target breathing rate for a second scenario;
Fig. 10 shows an example of the user breathing rate and the target breathing rate for a third scenario;
Fig. 11 shows an example of the user breathing rate and the target breathing rate for a fourth scenario; and
Fig. 12 shows a positive airway pressure support system.

DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs are merely schematic and are not drawn to scale. It should also be understood that the same reference numerals are used throughout the Figs to indicate the same or similar parts.

The invention provides a control system for controlling a pressure applied by a pressure source of a positive airway pressure support system. A progressive increase is provided in the pressure applied during a fall asleep time period of a user of the system. A pressure modulation is applied during the progressive increase. The modulation is for promoting a target breathing rate of the user.

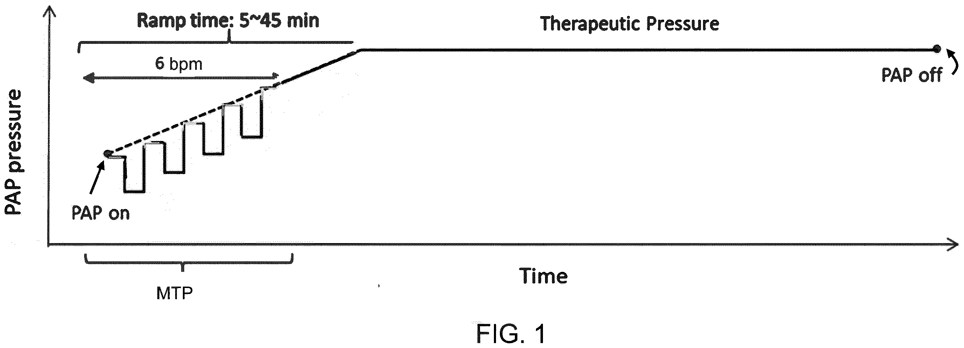

Fig. 1 shows a graph of pressure applied by a positive airway pressure (PAP) system over time, using a most basic implementation of the invention. The pressure increases along a ramp from a low level such as 4cmH2O = 400Pa at the start of the ramp to a treatment (therapeutic) pressure at the end of the ramp such as 10cmH20 = 1kPa. As shown, the ramp time has a duration for example in the range 5 minutes to 45 minutes and it is a fall asleep period, when the user of the PAP system is trying to fall asleep.

During part or all of the ramp period, there is a modulation time period (MTP). This is a period during which guidance is provided for slow-paced breathing. It for example has a duration of 5 to 20 minutes, and is used until the user falls asleep. If a user cannot fall asleep during the MTP, the PAP will continue following a ramp or treatment (therapeutic) pressure without pressure modulation guided slow-paced breathing.

The slow-paced breathing guidance is provided as a modulation to the ramp pressure, so that there is an alternation between periods of higher and lower pressure. A first, higher, pressure is used during times when the user is guided to perform an inhalation period of a breathing cycle, and there is a drop from the higher pressure to a second, lower, pressure for an exhalation period of the breathing cycle. In the example shown, the pressure is modulated so that the original ramp is the maximum pressure, and the modulation is thus a shaped square waveform reduction in pressure as an example, subtracted from the ramp profile. However, the modulation in pressure could instead be an increase above the ramp, or an increase and decrease compared to the ramp.

The resulting waveform has a characteristic frequency (corresponding to the square wave profile as an example), and it corresponds to a target breathing rate, i.e. number of breaths per minute. In the most simple example of Fig. 1, the waveform sets a fixed and constant target breathing rate of 6 bpm. This is an example of a desired breathing rate for slow-paced breathing.

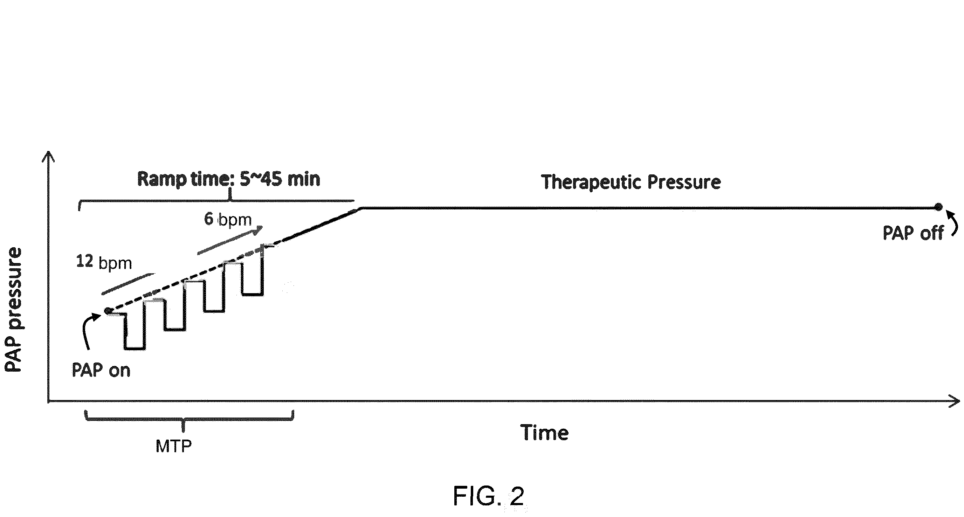

Fig. 2 shows a graph of pressure applied by a positive airway pressure (PAP) system over time, using a more sophisticated implementation of the invention.

The target breathing rate, set by the modulation of the pressure applied by the PAP system, for example, evolves from a starting value of 12 bpm (i.e., a calculated breathing rate based on the user's actual breathing rate; if a user has a higher breathing rate, the modulation will start at a higher breathing rate) to 6 bpm (i.e., a target breathing rate). This provides a reduction in the breathing rate which the user is trying to follow.

The way different target breathing rates may be set and how they evolve over time is discussed in more detail below.

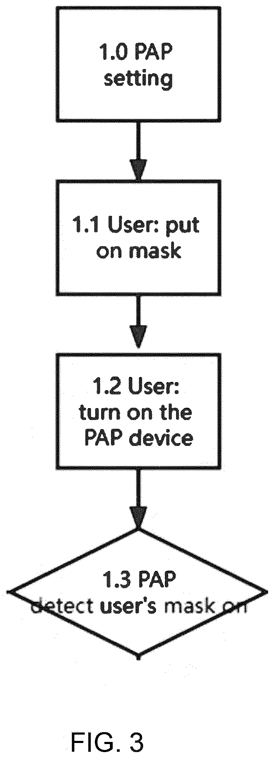

Fig. 3 shows a first part of a flow chart to explain the operation of one example of a pressure control system.

In step 1.0, the parameters of the PAP therapy are set, including the treatment pressure (P_treatment), the target slow-paced breathing rate ($\alpha$, e.g., 6 bpm), the preferred slow-paced breathing category (if any), and the MTP time (T_preset). This step is conducted before the first PAP usage of the prescribed PAP therapy and does not require frequent change.

Each treatment starts with the user putting on the device in step 1.1.

In step 1.2, the user turns on the PAP device. The pressurized air is then generated by the PAP device and is delivered to a connected tube and mask. The device detects the change of pressure indicating that the mask has been put on in step 1.3 and then initiates the preset PAP therapy.

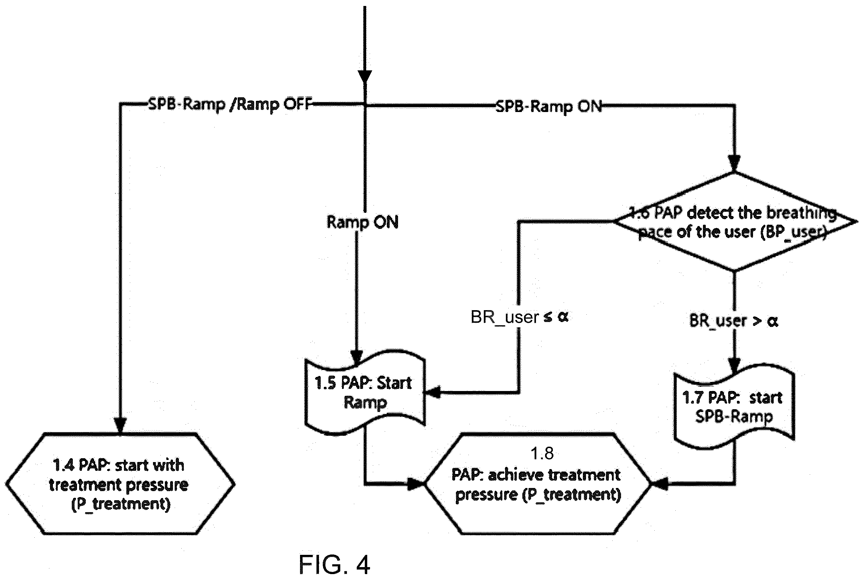

Fig. 4 shows different overall system modes that may be enabled, as a flow chart which follows from Fig. 3.

In step 1.4, if the slow-paced breathing function is set to OFF and the pressure ramp function is set to OFF then the preset PAP therapy will immediately start with P_treatment (with no ramp and no slow-paced breathing guidance). This may be considered to comprise a first overall system mode without the progressive pressure increase.

If the pressure ramp function is set as ON, the PAP delivers pressurized air from a low setting (e.g., 4 cmH2O), gradually ramping up to the set pressure P_treatment.

If the slow-paced breathing function is not turned ON, the conventional smooth (or stepped) pressure ramp is followed in step 1.5. In this step, the PAP system delivers pressurized air using the ramp profile explained above. This may be considered to be a second overall system mode with the progressive pressure increase but without the pressure modulation for slow-paced breathing guidance.

If the slow-paced breathing function is ON, the breathing rate of the user is detected in step 1.6. The detected breathing rate BR_user is compared with the desired breathing rate $\alpha$. This is a target breathing rate of the user, and is the pace realized by the PAP device by delivering the stepped lower and higher pressure levels as shown in Figs 1 and 2.

If the detected breathing rate detected BR_user is higher than α, the pressure ramp with SPB modulation is started in step 1.7. The PAP system delivers pressurized air in the SPB mode, from the low initial setting gradually ramping up to the treatment P treatment and with modulation of the pressure ramp to provide breathing guidance.

The treatment pressure is reached in step 1.8 at the end of the pressure ramp.

As explained above and shown in Figs 1 and 2, there are different possible modes for the slow-paced breathing function.

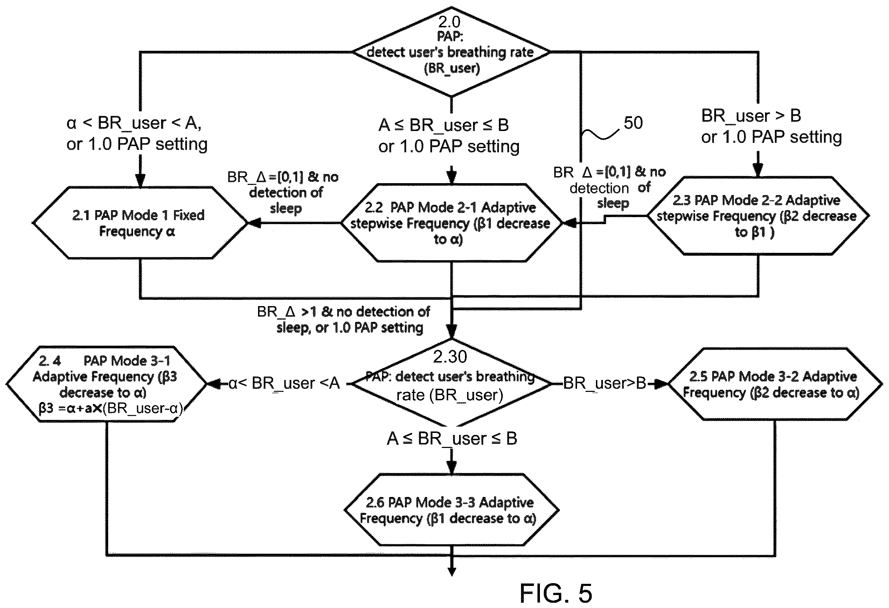

Fig. 5 shows a first part of a flow chart to explain the different steps of the slow-paced breathing function. In particular, Fig. 5 shows three SPB modes: fixed frequency, adaptive stepwise frequency, and adaptive (dynamic) frequency.

The guided target breathing rate used in the various SPB modes is based on the user's breathing rate (BR user). BR_user can be measured by sensors such as a pressure sensor installed in the PAP device.

In a first step 2.0 the initial breathing rate BR_user of the user is measured. In this particular example, the user is placed into one of three categories based on two thresholds A and B for their initial (i.e., at rest) breathing rate (or the user can also manually choose a category in spite of the user's breathing rate BR user).

A first category is a slow breather, where the breathing rate is between the target slow-paced breathing rate α (or other setting provided in step 1.0 of Fig. 3) and a first threshold A. As discussed below, this category of user follows a fixed frequency program.

A second category is a medium paced breather, where the breathing rate is between the first threshold A and a higher second threshold B. As discussed below, this category of user follows an adaptive stepwise frequency program.

A third category is a fast paced breather, where the breathing rate is above the second threshold B. As discussed below, this category of user also follows an adaptive stepwise frequency program but with more steps before the target breathing rate reaches the desired end breathing rate.

The adaptive (dynamic) frequency mode can be used when the user is failing to fall asleep after an adaptive stepwise frequency as discussed further below. The user may also choose to start with the adaptive frequency mode.

The threshold A is for example the lower bound of a normal breathing rate and the threshold B is the upper bound of a normal breathing rate. For example, this normal breathing rate may be 6 to 12 bpm.

As shown in Fig. 5, the user may choose which mode to follow (regardless of their breathing rate), by making a selection in step 1.0 of Fig. 3. This is what is meant by "or 1.0 PAP setting" in Fig. 5.

Line 50 illustrates that the adaptive (dynamic) frequency mode may be initiated manually by the user, thereby passing directly to step 2.30.

The initiation of the slow-paced breathing function can be conducted manually or automatically.

For a manual start, the SPB mode may be preselected manually in the device setting before the PAP therapy (in step 1.0). The user can start with the fixed frequency mode, adaptive stepwise frequency, or adaptive (dynamic) frequency and continue the preselected mode until the end of the SPB function, no matter which breathing category the user has.

For the automatic start, the SPB mode is selected automatically before the PAP therapy based on the user's real-time (initial) breathing rate (BR_user) and hence their breathing rate category.

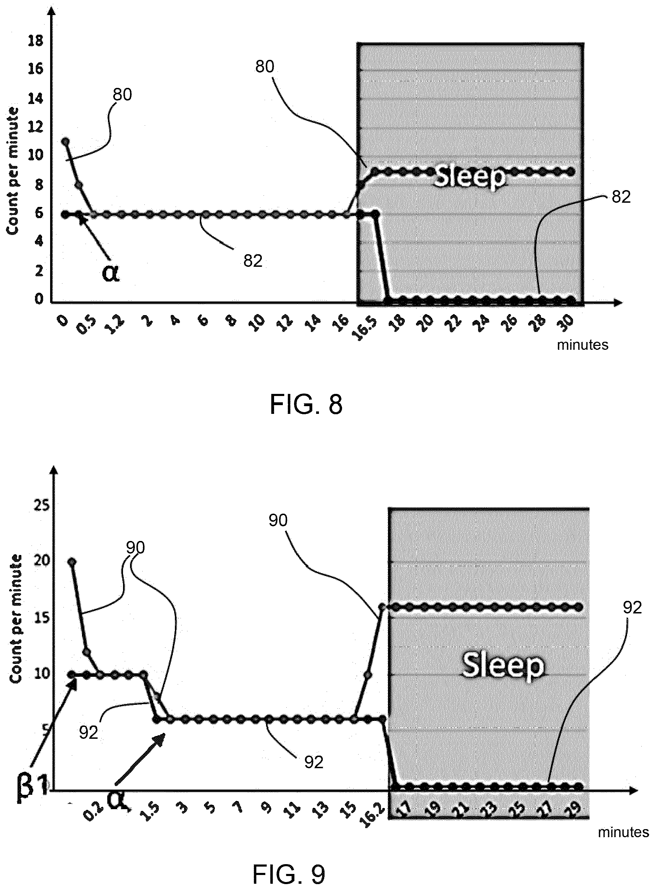

When the breathing rate BR_user falls into the slow breather category, the fixed frequency (mode 1) is selected in step 2.1: The PAP system starts the pressure ramp and initiates the SPB mode with a preset target frequency α. This is the desired slow-paced breathing rate. The PAP pressure waveform example is then as shown in Fig. 1. For example, a user with a BR_user of 11 bpm is made to follow a fixed frequency of 6 bpm in the example shown in Fig. 8.

When the BR_user falls into the medium breather category, the adaptive stepwise frequency mode is selected in step 2.2. The PAP system starts the pressure ramp and initiates the SPB mode, with an initial target breathing rate β1. This may be selected based on the initial user breathing rate BR_user. This target β1 continues for a short time while checking if the user can follow the target β1. If the user can follow the target β1, then the process proceeds to step 2.1 so that the fixed frequency mode is used with the target set as the desired final slow-paced breathing rate α. In the adaptive stepwise frequency mode, the target β1 changes to the desired final slow-paced breathing rate α immediately without a gradually changing process.

When the breathing rate BR_user falls into the fast breather category, the adaptive stepwise frequency is also selected but at a higher initial target in step 2.3. The PAP system starts the pressure ramp and initiates the SPB mode with a higher preset target frequency β2. This target β2 continues for a short time while checking if the user can follow the target β2. If the user can follow the target β2, then the process proceeds to step 2.2 so that the new lower target β1 is set. Once the target β1 can be followed, the process again proceeds to step 2.1. For the change from the target β2 to the target β1 and the change from the target β1 to the desired final slow-paced breathing rate α, those two changes occur immediately without a gradually changing process.

The values β1 and β2 depend on the initial breathing rate of the user. For example:

$$\beta1 = \alpha + a \times (BR\_user - A)$$

$$\beta2 = \alpha + b \times (BR\_user - A)$$

$$a \in (0, 1), b \in (0, 1), a < b.$$

The value (BR user- A) is a measure of the amount by which the user's breathing rate exceeds the lowest bound of the normal breathing rate range.

By way of example, a=0.5, b=0.7, and these values are used in the examples of Figs 8 and 9. This makes sure that a user with a higher breathing rate can start with a relatively higher breathing rate, so the user can accommodate to the slower breathing rate more comfortably.

When a user cannot follow these two modes (fixed frequency or stepwise frequency) and is still awake, the adaptive (dynamic) frequency mode is started. This uses a larger number of smaller reductions in target breathing rate. A progressive and gradual smooth change in target breathing rate is thereby implemented.

As shown in Fig. 5, the steps 2.1, 2.2 and 2.3 are ended and the process advances to step 2.30 if there is no detection of sleep and a difference value $BR\_\Delta$ (between the current breathing rate and the target breathing rate) exceeding a threshold, such as 1 bpm.

The current breathing rate is detected in step 2.30.

If the user's breathing rate is below the threshold A (but above the desired breathing rate $\alpha$), the target breathing rate starts at a new target value $\beta3$ and then decreases gradually to $\alpha$, in step 2.4. For example:

$$\beta3 = \alpha + a \times (BR\_user - \alpha)$$

If the user's breathing rate is between the thresholds A and B, the target breathing rate starts at a value $\beta1$ and then decreases gradually to $\alpha$, in step 2.6.

If the user's breathing rate is above the threshold B, the target breathing rate starts at a value $\beta2$ and then decreases gradually to $\alpha$, in step 2.5.

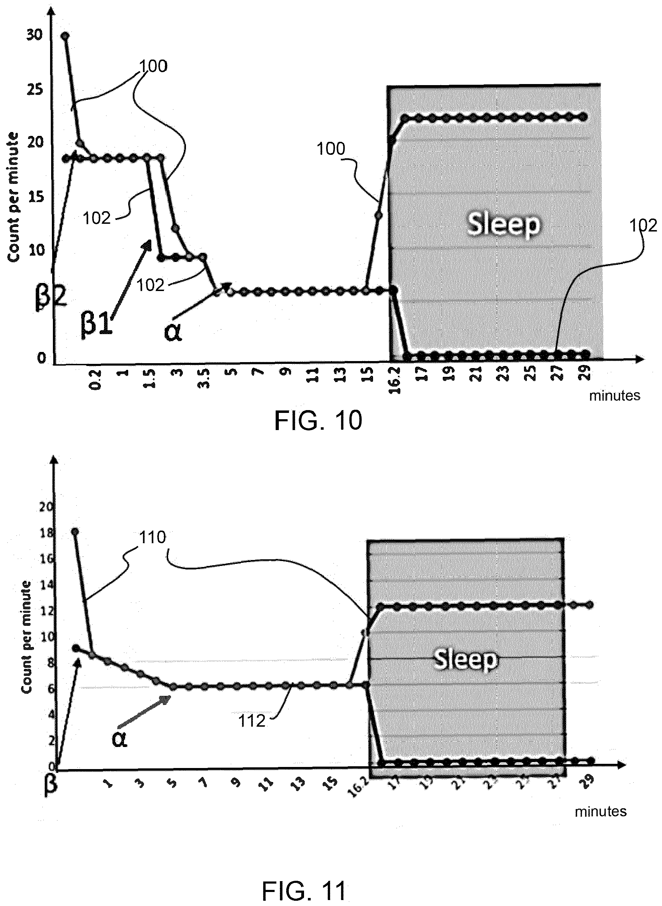

The gradual decreasing process used in this third mode for example has constant rate of decrease of the target breathing rate, such as a decrease rate of 0.5 bpm/minute (see Fig. 11).

Thus, the adaptive frequency mode reduces the target breathing rate gradually down until it is the desired end breathing rate for slow-paced breathing, i.e., $\alpha$. The starting point depends on the current breathing rate.

The slow-paced breathing guidance ends if the user has fallen asleep.

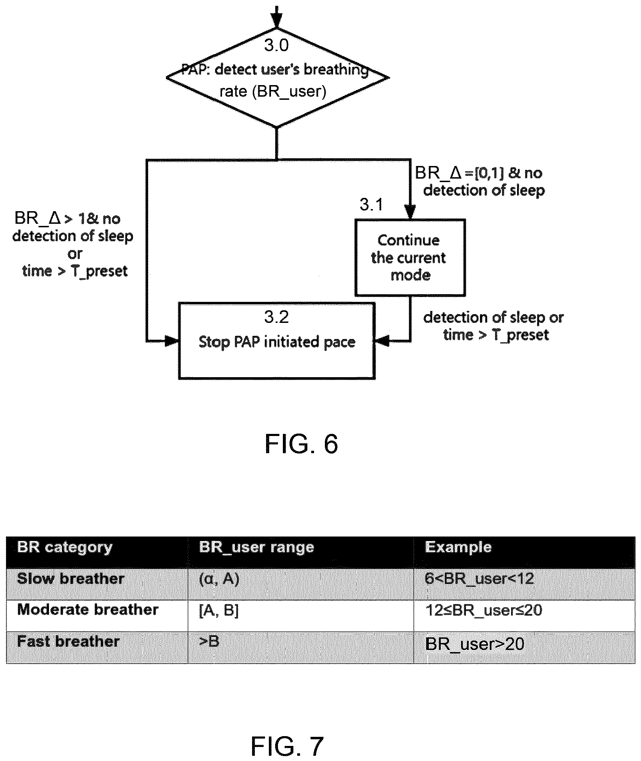

Fig. 6 shows how it is determined when to stop the slow-paced breathing guidance for situations where the user has not fallen asleep. Fig. 6 follows on from Fig. 5.

After performing the SPB mode for a fixed time, if the user can follow the SPB mode (for example if $BR\_\Delta$ is in the range 0 to 1) and is still awake, the SPB will continue till reaching T_preset. This is shown as step 3.1. If the user can follow the SPB mode and has still not fallen asleep when the maximum time T_preset is reached, then the SPB mode is stopped in step 3.2.

If the user cannot follow the SPB mode (for example if $BR\_\Delta > 1$) and is still awake, the SPB will also stop in step 3.2.

If the user cannot follow the SPB mode (for example if $BR\_\Delta > 1$) but nevertheless falls asleep, the SPB will stop in step 3.2.

As explained above, the process advances to step 3.2 if the time of the slow-paced breathing exceeds a threshold T_preset. This threshold T_preset can be defined at the beginning of the therapy. The maximum time of SPB and hence T_preset should be shorter than the ramp period.

As mentioned above, the determination of whether a target breathing rate can be followed is based on the value $BR\_\Delta$. After initiating each SPB mode, $BR\_\Delta$ is computed based on the difference between the guided rate and BR_user within a fixed time. If $BR\_\Delta = [0,1]$, the user can follow the current SPB mode, otherwise not.

For the detection of sleep, falling asleep during PAP therapy is detected in known manner based on the changes of respiratory rate, flow and event (i.e., large standard deviation of breathing rate, lower flow and detection of apnea event).

Fig. 7 shows the breathing rate categories explained above.

A slow breather is in the range $\alpha$ (e.g., 6) to A (e.g., 12) (if their rest rate is below $\alpha$ they do not need SPB guidance).

A medium breather is in the range A (e.g., 12) to B (e.g., 20).

A fast breather is above B (e.g., 20).

Fig. 8 shows an example of the user breathing rate (plot 80) and the target breathing rate (plot 82) for a scenario in which the user's breathing rate BR user is detected as 11 bpm (a slow breather in Fig. 7). The SPB mode is automatically set with a breathing target $\alpha=6$ bpm. The user will try to slow the breathing rate until they reach 6 bpm and follow 6 bpm during their awake time. When the user falls asleep, their breathing rate increases. The conditions of not following the SPB target breathing rate and falling asleep are detected by the device, and when sleep is detected the SPB mode stops and the therapy continues, following the non-modulated ramp. The target breathing rate may be considered to be zero when the SPB mode is ended.

Fig. 9 shows an example of the user breathing rate (plot 90) and the target breathing rate (plot 92) an alternative scenario in which the user's breathing rate BR_user is detected as 20 bpm (a moderate breather in Fig. 7). The SPB mode is automatically set with $\beta1=10$ bpm. The user will try to slow the breathing rate until they reach 10 bpm (becoming a slow breather in Fig. 7). Afterwards, the SPB target is automatically set with $\alpha=6$ bpm. The user will try to slow the breathing rate until they reach 6 bpm and follow 6 bpm during awake time. When the user falls asleep, his/her breathing rate increases.

and the SPB mode stops. The target breathing rate may again be considered to be zero when the SPB mode is ended.

Fig. 10 shows an example of the user breathing rate (plot 100) and the target breathing rate (plot 102) for an alternative scenario in which the user's breathing rate BR_user is detected as 30 bpm (fast breather in Fig. 7), the SPB is automatically set with breathing target β2=19 bpm. The user will try to slow the breathing rate until they reach 19 bpm (becoming a moderate breather in Fig. 7). Then, the SPB is automatically set with β1=9 bpm. The user will try to slow the breathing rate until they reach 9 bpm (becoming a slow breather). Afterwards, the SPB is automatically set with breathing target α=6 bpm. The user will try to slow the breathing rate until they reach 6 bpm and follow 6 bpm during awake time.

Fig. 11 shows an example of the user breathing rate (plot 110) and the target breathing rate (plot 112) when the adaptive (dynamic) frequency mode is used. In this case, the user has manually chosen to start with adaptive (dynamic) frequency without going through the fixed and adaptive stepwise frequency modes.

The user's breathing rate BR user is detected as 18 bpm (a moderate breather in Fig. 7), the SPB target breathing rate is automatically set with β1=9 bpm. The user will try to slow the breathing rate until they reach 9 bpm (becoming a slow breather in Fig. 7). Then, the SPB incrementally decreases from 9 bpm until the user reaches 6 bpm (α) overtime (e.g., decrease at a rate of 0.5 bpm /minute).

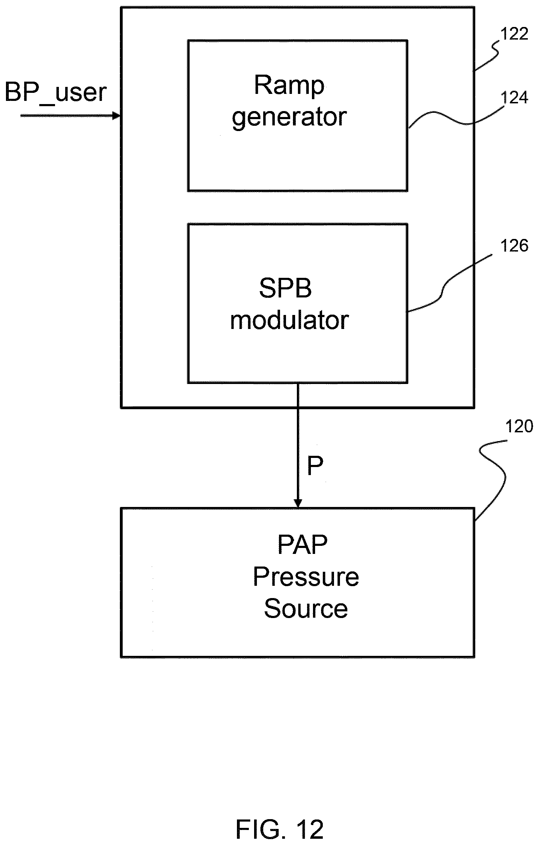

Fig. 12 shows a positive airway pressure support system, comprising a PAP pressure source 120 and a control system 122 for implementing the control method explained above. The control system 122 has a ramp generator 124 and a pressure modulator 126.

The invention provides a method of controlling a pressure applied by a pressure source of a positive airway pressure support system, to apply the progressive increase in the pressure during the fall asleep time period and to apply the pressure modulation. The method is preferably implemented in software which controls the pressure source of the positive airway pressure system. The software comprises a computer program, i.e., computer program code means, which is run by a processor forming part of the control system of the positive airway pressure system. The computer program is for example embodied as a computer program medium (i.e., a memory device).

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a processor may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

**Claims**

1. A control system (122) for controlling a pressure applied by a pressure source of a positive airway pressure support system, wherein the control system comprises a controller (124, 126) which is configured:

    to apply a progressive increase in the pressure applied during a fall asleep time period of a user of the system; and
    to apply a pressure modulation during the progressive increase, wherein the modulation is for promoting a target breathing rate of the user.

2. The system of claim 1, wherein the controller is further configured to receive a current breathing rate of the user from a breathing rate sensor.

3. The system of claim 2, wherein the controller is configured to apply a pressure modulation for promoting a target breathing rate which depends on the current breathing rate of the user.

4. The system of claim 3, wherein there is a desired breathing rate (α) to be reached by the end of the progressive increase in the pressure applied, and wherein the controller is configured:

    in a first mode, if the current breathing rate is below a first threshold (A) and above the desired breathing rate, to set the desired breathing rate (α) as the target breathing rate; and
    in a second mode if the current breathing rate is above a threshold (A), to set the target breathing rate to a target (β1) above the desired breathing rate (α) and to change to the first mode when the current breathing rate reaches the target (β1).

5. The system of claim 4, wherein the second mode comprises a set of thresholds (A, B), wherein if the current breathing rate is above a highest threshold (B), the controller is configured to set the target breathing rate to a highest target (β2), and to change to a lower target (β1) when the current breathing rate reaches the current target (β2), and to change to the first mode when the current breathing rate reaches a lowest target (β1) of the second mode.

6. The system of claim 4 or 5, wherein one or more target breathing rates are set in dependence on an initial or default breathing rate of the user.

7. The system of claim 4, 5 or 6, wherein the controller is configured:
   in a third mode, if the user cannot follow the one or more targets (β1, β2) of the second mode and is still awake, to reduce the target breathing rate gradually from a starting value towards to the desired breathing rate (α).

8. The system of claim 7, wherein the starting value in the third mode comprises:

   if the current breathing rate is below the first threshold (A) and above the desired breathing rate (α), a target (β3) which is a function of the desired breathing rate (α) and the breathing rate of the user (BR user) and is greater than the desired breathing rate; and
   if the current breathing rate is above the first threshold (A), the target (β1, β2) above the desired breathing rate (α) of the second mode.

9. The system of any one of claims 2 to 8, wherein the controller is configured to determine a difference (BR_Δ) between the current breathing rate of the user and the target breathing rate thereby to determine if the user can follow the target breathing rate, and:

   before a time period threshold has been reached, if the user cannot follow the target breathing rate sufficiently well and has not fallen asleep, to end the pressure modulation; and
   before the time period threshold has been reached, if the user can follow the target breathing rate sufficiently well but has not fallen asleep, to continue the pressure modulation; and
   to end the pressure modulation when the time period threshold has been reached or after the user has fallen asleep.

10. The system of any one of claims 1 to 9, wherein the controller is configured to implement:

    a first overall system mode without the progressive increase;
    a second overall system mode with the progressive increase but without the pressure modulation; and
    a third overall system mode with the progressive increase and with the pressure modulation.

11. The system of any one of claims 1 to 10, wherein the controller is configured to select one or more target breathing rates for the pressure modulation in dependence on a category of user, wherein the category relates to a default breathing rate of the user.

12. The system of any one of claims 1 to 11, wherein the pressure modulation comprises a first pressure for an inhalation period of a breathing cycle and a drop from the first pressure to a second pressure for an exhalation period of the breathing cycle.

13. A positive airway pressure support system, comprising:

    a pressure source; and
    the control system of any one of claims 1 to 12 for controlling a pressure applied by the pressure source.

14. A method of controlling a pressure applied by a pressure source of a positive airway pressure support system, comprising:

    applying a progressive increase in the pressure applied during a fall asleep time period of a user of the system; and
    applying a pressure modulation during the progressive increase, wherein the modulation is for promoting a target breathing rate of the user.

15. A computer program comprising computer program code means which is adapted, when said program is run on the controller of the positive airway pressure support system of claim 13, to implement the method of claim 14.

Therapeutic Pressure

PAP off

Ramp time: 5~45 min

6 bpm

PAP on

MTP

PAP pressure

Time

FIG. 1

10

FIG. 2

FIG. 3

FIG. 4

FIG. 5

FIG. 6

| BR category | BR_user range | Example |
|---|---|---|
| Slow breather | $(\alpha, A)$ | $6 < BR\_user < 12$ |
| Moderate breather | $[A, B]$ | $12 \leq BR\_user \leq 20$ |
| Fast breather | $> B$ | $BR\_user > 20$ |

FIG. 7

FIG. 8

FIG. 9

FIG. 10

FIG. 11

BP_user →

Ramp
generator

122

124

SPB
modulator

126

P

PAP
Pressure
Source

120

FIG. 12

Europäisches
Patentamt

European
Patent Office

Office européen
des brevets

# EUROPEAN SEARCH REPORT

**Application Number**

**EP 22 20 6553**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/078255 A1 (BOWMAN BRUCE R [US] ET AL) 26 March 2009 (2009-03-26) * paragraphs [0002], [0033], [0041], [0043], [0044], [0049], [0051], [0053] – [0063], [0069] – [0073], [0082] – [0101], [0111], [0113]; figures * | 1-15 | INV. A61M16/00 |
| X | US 2015/250963 A1 (RAMANAN DINESH [AU] ET AL) 10 September 2015 (2015-09-10) * paragraphs [0115], [0116], [0190], [0225] – [0253], [0263]; figures * | 1,2,10, 12-15 | |
| X | US 2008/092894 A1 (NICOLAZZI PASCAL [FR] ET AL) 24 April 2008 (2008-04-24) * paragraphs [0004], [0031] – [0036]; figure 1C * | 1-15 | |
| X | US 2017/014585 A1 (GERRED ANDREW GORDON [NZ] ET AL) 19 January 2017 (2017-01-19) * paragraphs [0038], [0041] – [0044], [0050] – [0065]; figures * | 1-15 | |
| | | | TECHNICAL FIELDS SEARCHED (IPC) |
| A | US 2006/249149 A1 (MEIER JOERG [DE] ET AL) 9 November 2006 (2006-11-09) * paragraphs [0058], [0059], [0147] – [0167]; figures * | 1-15 | A61M |
| A | EP 2 994 182 B1 (KONINKLIJKE PHILIPS NV [NL]) 26 August 2020 (2020-08-26) * paragraphs [0044], [0045]; figures * | 1-15 | |

The present search report has been drawn up for all claims

| Place of search | Date of completion of the search | Examiner |
|---|---|---|
| Munich | 15 September 2023 | Cametz, Cécile |

EPO FORM 1503 03.82 (P04C01)

## ANNEX TO THE EUROPEAN SEARCH REPORT
## ON EUROPEAN PATENT APPLICATION NO.

EP 22 20 6553

This annex lists the patent family members relating to the patent documents cited in  the above-mentioned European search report.
The members are as contained in the European Patent Office EDP file on
The European Patent Office is in no way liable for these particulars which are merely given for the purpose of information.

15-09-2023

| Patent document cited in search report | | Publication date | Patent family member(s) | | Publication date |
|---|---|---|---|---|---|
| US 2009078255 | A1 | 26-03-2009 | EP | 2195057 A1 | 16-06-2010 |
| | | | US | 2009078255 A1 | 26-03-2009 |
| | | | WO | 2009039527 A2 | 26-03-2009 |
| US 2015250963 | A1 | 10-09-2015 | NONE | | |
| US 2008092894 | A1 | 24-04-2008 | FR | 2906474 A1 | 04-04-2008 |
| | | | US | 2008092894 A1 | 24-04-2008 |
| | | | WO | 2008039979 A2 | 03-04-2008 |
| US 2017014585 | A1 | 19-01-2017 | AU | 2015242215 A1 | 20-10-2016 |
| | | | AU | 2020239789 A1 | 29-10-2020 |
| | | | CA | 2944631 A1 | 08-10-2015 |
| | | | CN | 106456922 A | 22-02-2017 |
| | | | CN | 110975092 A | 10-04-2020 |
| | | | EP | 3113819 A1 | 11-01-2017 |
| | | | EP | 3741417 A1 | 25-11-2020 |
| | | | EP | 4101487 A1 | 14-12-2022 |
| | | | FR | 3019468 A1 | 09-10-2015 |
| | | | JP | 6660886 B2 | 11-03-2020 |
| | | | JP | 7023995 B2 | 22-02-2022 |
| | | | JP | 2017510362 A | 13-04-2017 |
| | | | JP | 2020110597 A | 27-07-2020 |
| | | | JP | 2022070942 A | 13-05-2022 |
| | | | SG | 10201808623X A | 29-11-2018 |
| | | | SG | 11201608150V A | 28-10-2016 |
| | | | US | 2017014585 A1 | 19-01-2017 |
| | | | US | 2021187218 A1 | 24-06-2021 |
| | | | WO | 2015150997 A1 | 08-10-2015 |
| US 2006249149 | A1 | 09-11-2006 | EP | 1605998 A1 | 21-12-2005 |
| | | | JP | 2006520227 A | 07-09-2006 |
| | | | US | 2006249149 A1 | 09-11-2006 |
| | | | WO | 2004082751 A1 | 30-09-2004 |
| EP 2994182 | B1 | 26-08-2020 | CN | 105209099 A | 30-12-2015 |
| | | | EP | 2994182 A1 | 16-03-2016 |
| | | | JP | 2016523586 A | 12-08-2016 |
| | | | US | 2016089509 A1 | 31-03-2016 |
| | | | WO | 2014181244 A1 | 13-11-2014 |

EPO FORM P0459

For more details about this annex : see Official Journal of the European Patent Office, No. 12/82